# FASCICULE DE BREVET EUROPEEN

(11) **EP 2 693 998 B1**
(45) Date de publication et mention de la délivrance du brevet: **01.03.2017**
(21) Numéro de dépôt: 12711902.2
(22) Date de dépôt: 05.04.2012
(51) Int. Cl.: A61F 9/007, A61N 7/02

(54) **DISPOSITIF DE THERAPIE OCULAIRE PAR ULTRASONS A REFLECTEUR**
VORRICHTUNG FÜR OKULARE ULTRASCHALLTHERAPIE MIT REFLEKTOR
DEVICE FOR OCULAR ULTRASOUND THERAPY HAVING A REFLECTOR

(30) Priorité: 05.04.2011 FR 1152933
(43) Date de publication de la demande: 12.02.2014
(73) Titulaire: Eye Tech Care, 69140 Rillieux-la-Pape (FR); Institut National de la Santé et de la Recherche Médicale (INSERM), 75013 Paris (FR)
(72) Inventeur: ROMANO, Fabrizio, 01700 Beynost (FR); CHARREL, Thomas, 69003 Lyon (FR); LAFON, Cyril, 69780 Toussieu (FR); CHAPUIS, Philippe, 69480 Pommiers (FR); CHAPELON, Jean-Yves, 69100 Villeurbanne (FR)
(74) Mandataire: Regimbeau
(86) Numéro de dépôt international: PCT/EP2012/056254
(87) Numéro de publication internationale: WO 2012/136752

(56) Documents cités:
- WO-A-2010/094349
- WO-A1-2009/103721
- WO-A1-2011/020495
- US-A- 4 484 569
- US-A- 5 230 334

## Description

### DOMAINE DE L'INVENTION

La présente invention concerne le domaine technique général du traitement non invasif d'une pathologie oculaire.

Elle concerne plus particulièrement un dispositif et un procédé de génération d'ultrasons focalisés, plans ou défocalisé de haute ou faible intensité pour le traitement d'une telle pathologie oculaire tel qu'un glaucome.

### ARRIERE-PLAN DE L'INVENTION

Le glaucome est une neuropathie optique, c'est à dire un trouble du nerf optique, due à une pression intraoculaire (PIO) élevée.

L'oeil est une structure creuse composée de deux segments: le segment antérieur entre la cornée et le cristallin, est le segment postérieur entre le cristallin et la rétine. Le segment antérieur contient un liquide transparent appelé "l'humeur aqueuse."

L'humeur aqueuse est formée dans la chambre postérieure du segment antérieur de l'oeil par le corps ciliaire. Le liquide, qui est généré à un taux relativement constant, passe ensuite autour du cristallin, à travers l'ouverture pupillaire de l'iris et dans la chambre antérieure de l'oeil. L'humeur aqueuse est ensuite évacuée principalement à travers le trabéculum et le canal de Schlemm.

Lorsque l'humeur aqueuse n'est plus évacuée suffisamment rapidement, celle-ci s'accumule, ce qui induit une augmentation de la PIO. L'augmentation de la PIO compresse les axones dans e nerf optique et peut également compromettre la vascularisation du nerf optique. Une PIO élevée pendant une longue période peut induire une perte de vision totale.

La seule approche thérapeutique actuellement disponible pour traiter le glaucome consiste à réduire la pression intraoculaire :
- soit en améliorant le drainage humeur aqueuse,
- soit en réduisant la production d'humeur aqueuse.

On connaît différents dispositifs pour réduire la production d'humeur aqueuse basés sur le principe de cyclophotocoagulation. Les documents DE 44 30 720 et WO 02/38078 décrivent des dispositifs comprenant chacun un applicateur de génération d'énergie permettant de brûler une zone ponctuelle du corps ciliaire afin que le corps ciliaire produise moins d'humeur aqueuse. L'utilisateur positionne l'applicateur sur la partie blanche de l'oeil, en contact avec la sclère, selon une incidence perpendiculaire à la surface de l'oeil. Il génère ensuite un tir pour brûler une zone ponctuelle du corps ciliaire. L'utilisateur déplace ensuite l'applicateur pour brûler une zone suivante du corps ciliaire.

Un inconvénient de ces dispositifs est qu'ils ne permettent de traiter qu'une zone ponctuelle du corps ciliaire à la fois, de sorte qu'il est nécessaire de répéter les opérations de positionnement de l'applicateur et de tirer une pluralité de fois pour traiter toute la circonférence de l'oeil. Cette répétition des opérations de positionnement et de tir augmente le temps opératoire ainsi que le risque d'erreur.

Pour pallier ces inconvénients, le document WO 2009/103721 propose notamment un dispositif de traitement d'une pathologie oculaire comprenant un anneau présentant une partie proximale destinée à être en contact avec un oeil et une partie distale destinée à recevoir des moyens de génération d'ultrasons focalisés de haute intensité. Les moyens de génération d'ultrasons comprennent six transducteurs à profil concave en forme en segment de cylindre positionnés sur une couronne cylindrique.

Le document WO 2010/094349 décrit un dispositif comprenant un anneau et des moyens de génération d'ultrasons focalisés de haute intensité. Les moyens de générations peuvent comprendre en combinaison des transducteurs plans et un système de focalisation, tel qu'une lentille acoustique ou des réflecteurs acoustiques, s'étendant sous ou devant les transducteurs plans. Ce document relève de l'article 54(3) CBE et donc n'est pas pris en considération pour l'appréciation de l'activité inventive.

Ces dispositifs permettent le traitement du glaucome en un seul geste. Toutefois, la fabrication des moyens de génération d'ultrasons focalisés de haute intensité et notamment le positionnement des transducteurs sur le support cylindrique peut être difficile à mettre en oeuvre.

Un but de la présente invention est de proposer un dispositif plus facile à fabriquer que le dispositif décrit dans WO 2009/103721.

### BREVE DESCRIPTION DE L'INVENTION

A cet effet, l'invention propose un dispositif de traitement d'une pathologie oculaire selon la revendication 1. Ce dispositif comprend un anneau comportant une extrémité proximale destinée à être en contact avec un oeil d'un patient et une extrémité distale destinée à recevoir des moyens de génération d'ultrasons, remarquable en ce que le dispositif comprend en outre au moins un réflecteur incluant une face active concave en regard des moyens de génération d'ultrasons pour réfléchir et focaliser les ultrasons générés par les moyens de génération dans une région de l'oeil du patient.et les moyens de génération d'ultrasons comprennent au moins un transducteur comportant une face avant pour rayonner les ultrasons, la face avant de chaque transducteur s'étendant parallèlement à l'axe de révolution de l'anneau.

Des aspects préférés mais non limitatifs du dispositif selon l'invention sont les suivants :
- chaque réflecteur comprend une face active concave en regard des moyens de génération d'ultrasons pour réfléchir et focaliser les ultrasons dans une région de l'oeil du patient ;
- les moyens de génération d'ultrasons comprennent un transducteur tubulaire unique ;
- les moyens de génération d'ultrasons comprennent une pluralité de transducteurs, lesdits transducteurs étant positionnés sur des génératrices d'un tube ;
- chaque transducteur présente une forme en portion de cylindre ;
- chaque transducteur est plan ;
- le dispositif comprend en outre un support pour supporter les moyens de génération d'ultrasons et chaque réflecteur, les moyens de génération d'ultrasons étant positionnés dans une région centrale du support, et chaque réflecteur étant positionné dans une région périphérique du support ;
- le réflecteur comporte une face active concave torique ;
- le réflecteur comprend une pluralité d'éléments de réflexion, chaque élément de réflexion comportant une face active en portion de cylindre ;
- le dispositif comprend en outre un support pour supporter les moyens de génération d'ultrasons et chaque réflecteur, les moyens de génération d'ultrasons étant positionnés dans une région périphérique du support, et chaque réflecteur étant positionné dans une région centrale du support ;
- le réflecteur comporte une face active en forme de tronc de cône concave ou plan ou convexe ;
- les moyens de génération d'ultrasons présentent une forme tubulaire et le réflecteur présente une forme en tronc de cône concave, plan ou convexe, le diamètre des moyens de génération tubulaire étant supérieur au diamètre du réflecteur, les moyens de génération d'ultrasons et le réflecteur étant agencés de sorte que :
   - les axes de révolution du réflecteur et des moyens de génération d'ultrasons sont confondus,
   - les moyens de génération d'ultrasons entourent le réflecteur
   - la face interne des moyens de génération d'ultrasons tubulaire rayonnant les ultrasons est en regard de la face active du réflecteur.

### BREVE DESCRIPTION DES DESSINS

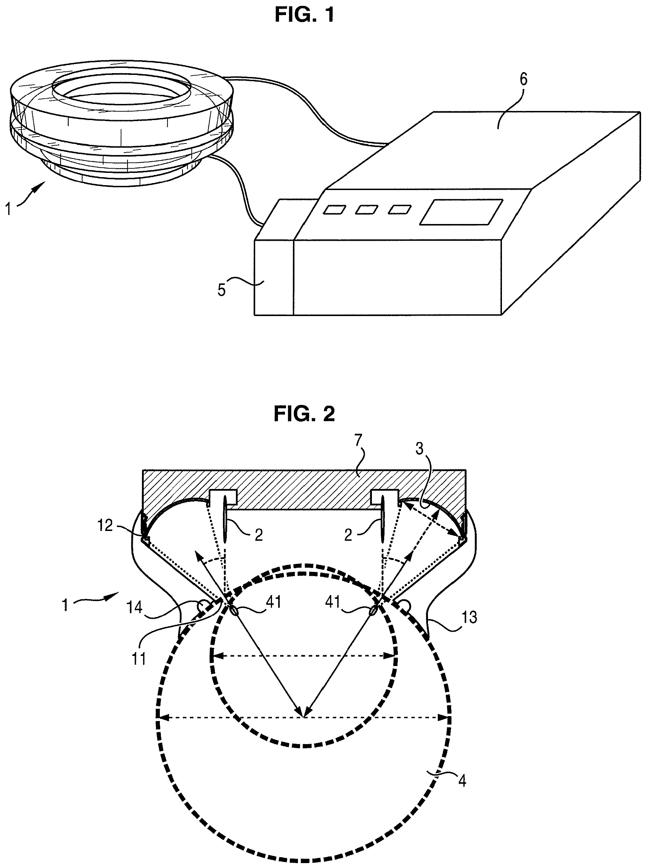

D'autres avantages et caractéristiques ressortiront mieux de la description qui va suivre de plusieurs variantes d'exécution, données à titre d'exemples non limitatifs, à partir des dessins annexés sur lesquels les figures 1 à 4 représentent différentes variantes du dispositif selon l'invention.

### DESCRIPTION DETAILLEE DE L'INVENTION

En référence aux figures 1 et 2, on a illustré un mode de réalisation du dispositif de traitement d'une pathologie oculaire.

Le dispositif comprend :
- un anneau 1,
- des moyens de génération d'ultrasons 2 pour générer des ultrasons, et
- un réflecteur 3 pour réfléchir, orienter et focaliser les ultrasons émis par les moyens de génération d'ultrasons 2.

### Anneau

L'anneau 1 consiste en un tronc de cône ouvert aux deux extrémités. La petite base 11 du tronc de cône constitue l'extrémité proximale de l'anneau 1, et la grande base 12 constitue l'extrémité distale de l'anneau 1.

L'extrémité proximale 11 est destinée à venir en contact avec un oeil 4 d'un patient. L'extrémité distale 12 de l'anneau 1 est destinée à recevoir les moyens de génération d'ultrasons 2.

En référence à la figure 1, l'extrémité proximale 11 du cône comprend une bride annulaire externe 13 apte à être appliquée sur la surface externe de l'oeil 4, à environ 2 mm du limbe, le limbe étant la jonction entre la cornée et lasclère.

Le bord proximal 11 du cône comporte également une gorge annulaire 14 reliée à un dispositif d'aspiration 5 par au moins une ouverture traversant le cône 1 et débouchant dans la gorge annulaire 14. Avantageusement, le dispositif d'aspiration 5 peut être contrôlé par une unité de commande 6.

Il est évident que le dispositif d'aspiration 5 peut être indépendant sans sortir du cadre de l'invention.

Le principe de fonctionnement du dispositif d'aspiration 5 est le suivant. Le cône 1 est appliqué sur l'oeil 4 du patient et le dispositif d'aspiration 5 est activé. Ceci induit la production d'une dépression dans la gorge annulaire 14 qui entraine une déformation de la conjonctive de l'oeil 4, cette déformation formant un joint torique dans la gorge annulaire 14.

Le cône 1 est alors étroitement lié à l'oeil 4 de sorte que le cône 1 suivra les micromouvements de l'oeil 4 au cours de la durée du traitement. Ceci permet d'assurer un maintien de la position centrée de l'appareil sur l'axe visuel.

Le matériau constituant l'anneau 1 peut être du silicone de qualité médicale. Ce matériau présente d'une part l'avantage d'être souple, et d'autre part l'avantage d'être compatible avec le contact de la conjonctive. Toutefois, il est évident que le tronc de cône 1 peut être constitué en n'importe quel matériau biocompatible connu par l'homme du métier tel que le PVC biocompatible.

### Moyens de générations d'ultrasons :

Les moyens de génération d'ultrasons 2 permettent de générer une énergie ultrasonore.

L'association des moyens de génération 2 et du réflecteur 3 permet d'obtenir une source d'ultrasons focalisés de haute intensité.

Les moyens de génération d'ultrasons 2 sont reliés à l'unité de commande 6. Celle-ci comporte un générateur de salve et des moyens pour spécifier les paramètres de la salve comme la fréquence, la puissance et la durée de chaque rafale, etc.

Le générateur de salve comprend au moins un générateur de signal sinusoïdal à une fréquence déterminée comprise entre 5 et 35 MHz, et de préférence entre 10 et 25 MHz, un amplificateur et un compteur électrique.

### Réflecteur

Le réflecteur 3 permet de réfléchir les ultrasons générés par les moyens de génération d'ultrasons 2.

Le réflecteur 3 comprend une face concave - dite « face active » - pour orienter et focaliser les ultrasons générés par les moyens de génération d'ultrasons 2 dans une zone de l'oeil 4, et notamment dans la région du corps ciliaire 41 de l'oeil 4. Plus précisément, chaque point de la face active concave est calculé itérativement en utilisant les lois de Snell-Descartes de sorte que les ultrasons réfléchis par ladite face active focalisent dans la région du corps ciliaire 41.

Le réflecteur 3 est dans un matériau permettant de réfléchir les ultrasons tel que du laiton.

### Exemples de modes de réalisation

On va maintenant décrire différents modes de réalisation du dispositif de traitement d'une pathologie oculaire par HIFU.

### Prototype 1

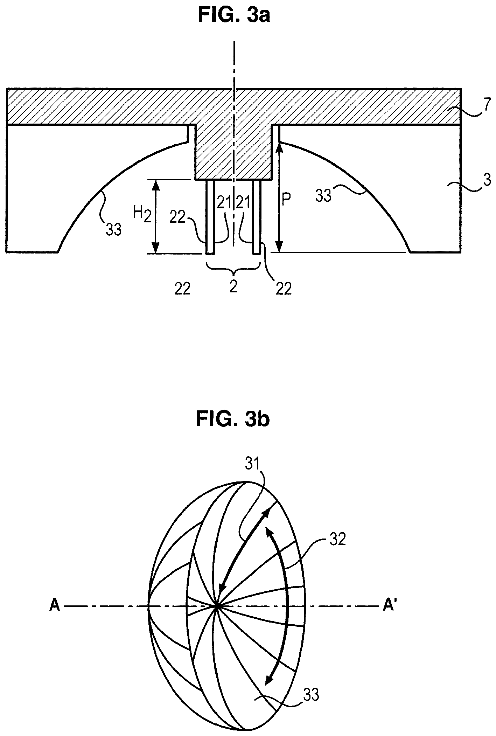

En référence à la figure 3, on a illustré un premier mode de réalisation du dispositif selon l'invention.

Le dispositif comprend l'anneau 1 décrit précédemment, des moyens de génération d'ultrasons 2 et un réflecteur 3.

Les moyens de génération d'ultrasons 2 consistent en un transducteur monobloc tubulaire. Le transducteur comprend une électrode intérieure 21 formant une face arrière du transducteur tubulaire (i.e. la face interne du tube) et une électrode extérieure 22 reliée à la masse formant une face avant du transducteur tubulaire (i.e. la face externe du tube) rayonnant les ultrasons générés. Dans le mode de réalisation illustré à la figure 3, le transducteur a un diamètre de 5 millimètres et une hauteur de 10 millimètres.

Le réflecteur 3 présente la forme d'une coupole percée en son sommet. Il comprend deux rayons de courbures :
- un premier rayon de courbure 31 dit méridionale passant par l'axe de révolution A-A' de la coupole 1 et
- un deuxième rayon de courbure 32 dit sagittal parallèle à l'axe de révolution A-A' de la coupole.

La face intérieure 33 concave de la coupole 3 forme une face active permettant de réfléchir les ultrasons générés par le transducteur tubulaire 2. Les dimensions du réflecteur 3 sont prévues suffisantes pour permettre le positionnement du transducteur 2 au centre du réflecteur 3. Notamment, la profondeur P de la coupole est supérieure ou égale à la hauteur H du transducteur 2.

Le réflecteur 3 et le transducteur 2 sont fixés sur un support 7 consistant en une couronne. Avantageusement, le diamètre extérieur du support 7 est supérieur ou égal au diamètre intérieur de l'extrémité distale 12 de l'anneau 1 pour permettre le positionnement du support 7 sur l'extrémité distale 12 de l'anneau 1. Le réflecteur 3 peut être intégré au support 7 de sorte que le support 7 et le réflecteur 3 sont constitués en une seule pièce. En variante, le réflecteur 3 et le support 7 peuvent être en plusieurs pièces.

Le transducteur 2 et le réflecteur 3 sont positionnés sur le support de sorte que leurs axes de révolution respectifs sont coaxiaux. Le transducteur 2 est positionné au centre du réflecteur 3. En d'autres termes, la portion utile de la face active 33 du réflecteur 3 entoure le transducteur tubulaire 2.

Le principe de fonctionnement du dispositif illustré à la figure 3 est le suivant. L'utilisateur positionne l'anneau 1 sur l'oeil 4 d'un patient. Une fois l'anneau 1 centré sur l'oeil 4, l'utilisateur active le dispositif d'aspiration 5. Il positionne ensuite le support 7 comportant les moyens de générations d'ultrasons 2 et le réflecteur 3 sur l'anneau 1.

L'utilisateur commande ensuite la génération d'ultrasons pour brûler le corps ciliaire 41. La face avant 22 du transducteur 2 rayonne les ultrasons qui se propagent radialement par rapport au réflecteur 3. Les ultrasons entrent en contact avec la face active 33 du réflecteur 3. Les ultrasons sont réfléchis par celle-ci et focalisés dans la zone du corps ciliaire 41 de l'oeil 4.

L'utilisation d'un transducteur tubulaire permet de faciliter la fabrication du dispositif de traitement d'une pathologie oculaire, ce type de transducteur étant résistant et facile à coller et à souder. Par ailleurs, ce dispositif permet de créer une lésion circulaire sur toute la circonférence du corps ciliaire 41, et ainsi de traiter l'ensemble du corps ciliaire 41 en une seule activation.

### Prototype 2

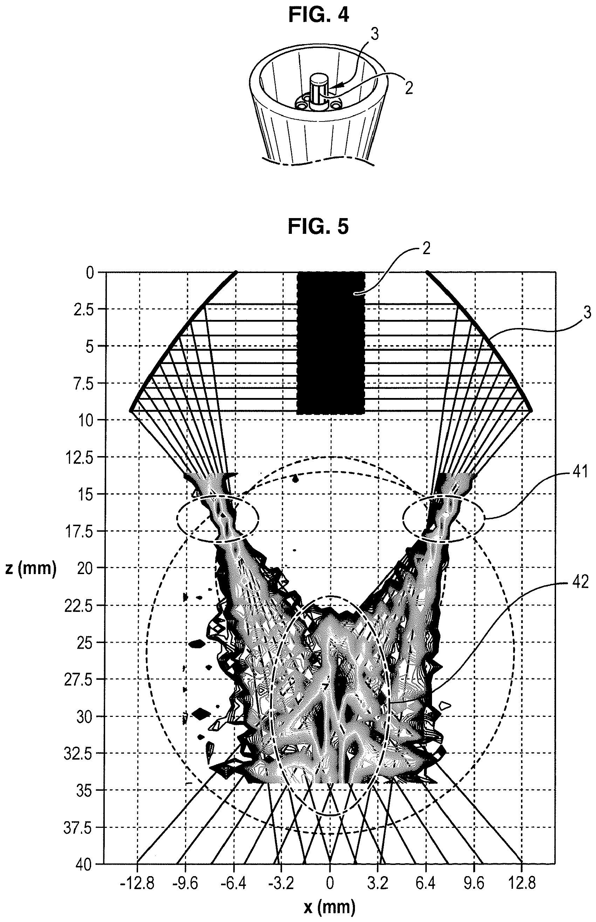

En référence à la figure 4 on a illustré une variante de réalisation du dispositif illustré à la figure 3. Ce dispositif comprend le même anneau 1 ainsi que le même réflecteur 3 que ceux décrits en référence à la figure 3.

Le dispositif illustré à la figure 4 diffère du dispositif de la figure 3 en ce que les moyens de génération d'ultrasons 2 comprennent huit transducteurs. Chaque transducteur est en forme de portions de cylindre de largeur 2 millimètres et de hauteur 6 millimètres. Ces transducteurs sont disposés les uns par rapport aux autres pour former un ensemble tubulaire.

Cet ensemble tubulaire de transducteurs peut être obtenu par exemple en sectorisant le transducteur monobloc illustré à la figure 3. La méthode de sectorisation peut consister à sectionner l'électrode extérieure du transducteur monobloc tubulaire parallèlement à son axe de révolution.

Ce dispositif permet de créer une pluralité de lésions en forme de portion de cercle au niveau du corps ciliaire.

### Modélisation des prototypes 1 et 2

La figure 5 illustre schématiquement une modélisation des dispositifs des figures 3 et 4. Comme on peut le constater, le dispositif permet l'obtention de deux zones de focalisation :
- une première zone de focalisation 41 dans une région du corps ciliaire due au rayon de courbure méridional,
- une deuxième zone de focalisation 42 dans une région du corps vitré due au rayon de courbure sagittal.

Pour ne pas provoquer d'effets indésirables dans la zone 42 du fond de l'oeil :
- la durée et la puissance des ultrasons générés par le transducteur tubulaire 2 du dispositif illustré à la figure 3 sont choisis suffisamment faibles pour éviter un échauffement trop important au niveau de la deuxième zone de focalisation 42,
- les transducteurs du dispositif illustré à la figure 4 sont activés successivement pour éviter un échauffement trop important au niveau de la deuxième zone de focalisation 42.

### Prototype 3

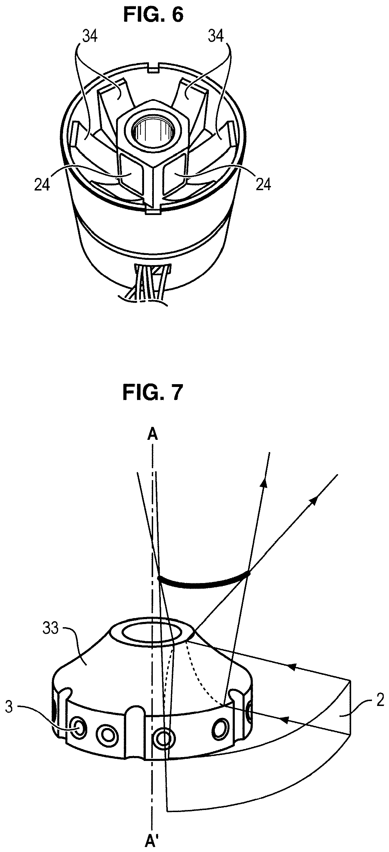

En référence à la figure 6, on a illustré un autre mode de réalisation du dispositif de traitement d'une pathologie oculaire.

Dans ce mode de réalisation, les moyens de génération d'ultrasons 2 comprennent six transducteurs rectangulaires plans 24. L'utilisation de transducteurs plans 24 est un avantage en termes de manipulation, de facilité de production et donc de coût. Ces transducteurs 24 sont positionnés les uns par rapport aux autres de sorte à former une alvéole de forme hexagonale en section.

Le dispositif comprend également six réflecteurs 34 en forme de portion de cylindre. Ces réflecteurs 34 entourent les moyens de génération d'ultrasons 2. Chaque réflecteur 34 est associé à un transducteur 24 plan respectif, la face active 33 du réflecteur 34 étant en regard de la face avant 22 de son transducteur 24 associé.

Le dispositif illustré à la figure 6 permet de créer six lésions linéaires au niveau du corps ciliaire 41.

La présence d'une pluralité de réflecteurs 34 en forme de portion de cylindre permet d'obtenir un ensemble de réflecteurs 34 présentant un unique rayon de courbure méridional 31.

La suppression du rayon de courbure sagittal 32 ainsi obtenue permet de s'affranchir de la deuxième zone de focalisation 42 des dispositifs illustrés aux figures 3 et 4.

### Prototype 4

En référence à la figure 7 on a illustré un autre mode de réalisation qui permet d'une part de limiter l'encombrement du dispositif illustré à la figure 5, et d'obtenir des lésions circulaire au niveau du corps ciliaire 41.

Dans ce mode de réalisation, les moyens de génération d'ultrasons 2 entourent le réflecteur 3.

Les moyens de génération d'ultrasons 2 sont en forme d'anneau. Ils peuvent être constitués d'un transducteur annulaire unique ou de plusieurs transducteurs en forme de portion de cylindre disposés les uns par rapport aux autres de sorte à former un ensemble annulaire. Dans tous les cas, les moyens de génération d'ultrasons 2 sont agencés pour générer des ultrasons se propageant radialement en direction de l'axe de révolution desdits moyens de génération d'ultrasons. Par exemple, dans le cas de moyens de générations d'ultrasons constitués d'un transducteur annulaire unique, la face interne de celui-ci est destinée à rayonner les ultrasons générés, la face externe étant reliée au point chaud.

La face active 33 du réflecteur 3 présente la forme d'un tronc de cône concave. Ainsi le réflecteur 3 comprend deux rayons de courbure : un rayon de courbure méridionale 31 lié à la concavité du réflecteur 3 et un rayon de courbure sagittal 32 lié à la symétrie de révolution du réflecteur 3.

Le principe de fonctionnement de ce dispositif est le suivant. Le (ou les) transducteur(s) 2 génèrent des ultrasons qui convergent vers l'axe de révolution A-A' du (ou des) transducteur(s) 2. Les ondes ultrasonores entrent en contact avec le réflecteur 3. La face active 33 du réflecteur 3 réfléchit les ondes ultrasonores en direction de l'oeil 4 du patient à traiter. Les ondes ultrasonores se propagent et focalisent dans la zone du corps ciliaire 41 de l'oeil 4 du patient du fait du rayon de courbure méridional 31. Après sa focalisation au niveau du corps ciliaire 41, le faisceau réfléchit continue de se propager vers l'extérieur de l'oeil 4 et focalise une deuxième fois à l'extérieur de l'oeil.

La forme en tronc de cône évasé - ou tronc de cône concave - du réflecteur induit donc deux focalisations : une première focalisation au niveau du corps ciliaire 41 et une deuxième focalisation à l'extérieur de l'oeil 4. Contrairement aux modes de réalisation illustrés aux figures 3 et 4, la deuxième focalisation est située à l'extérieur de l'oeil du fait des formes et agencement du réflecteur 3 et des moyens de génération d'ultrasons 2.

L'homme du métier aura compris que de nombreuses modifications peuvent être apportées au dispositif décrit ci-dessus sans sortir matériellement des nouveaux enseignements présentés ici.

Par exemple, dans les modes de réalisation décrits ci-dessus, la forme du réflecteur est adaptée pour focaliser les ultrasons, notamment grâce à la forme concave de la face active du réflecteur. En variante, la forme du réflecteur peut être adaptée pour défocaliser les ultrasons afin d'irradier une plus grande zone tissulaire sans focalisation, notamment grâce à une forme concave du réflecteur.

Il est donc bien évident que les exemples qui viennent d'être donnés ne sont que des illustrations particulières en aucun cas limitatives.

## Revendications

1. Dispositif de traitement d'une pathologie oculaire comprenant un anneau (1) comportant une extrémité proximale (11) destinée à être en contact avec un oeil (4) d'un patient et une extrémité distale (12) destinée à recevoir des moyens de génération d'ultrasons (2), le dispositif comprenant en outre au moins un réflecteur (3) incluant une face active (33) concave en regard des moyens de génération d'ultrasons (2) pour réfléchir et focaliser les ultrasons générés par les moyens de génération (2) dans une région (41) de l'oeil (4) du patient, et les moyens de génération d'ultrasons (2) comprenant au moins un transducteur comportant une face avant (22) pour rayonner les ultrasons, la face avant (22) de chaque transducteur s'étendant parallèlement à l'axe de révolution (A-A') de l'anneau (1).

2. Dispositif selon la revendication 1, dans lequel les moyens de génération d'ultrasons (2) comprennent un transducteur tubulaire unique.

3. Dispositif selon la revend cation 1, dans lequel les moyens de génération d'ultrasons (2) comprennent une pluralité de transducteurs, lesdits transducteurs étant positionnés sur des génératrices d'un tube.

4. Dispositif selon la revendication 3, dans lequel chaque transducteur présente une forme en portion de cylindre.

5. Dispositif selon la revendication 3, dans lequel chaque transducteur est plan.

6. Dispositif selon l'une quelconque des revendications 1 à 5, lequel comprend en outre un support (7) pour supporter les moyens de génération d'ultrasons (2) et chaque réflecteur (3), les moyens de génération d'ultrasons (2) étant positionnés dans une région centrale du support (7), et chaque réflecteur (3) étant positionné dans une région périphérique du support (7).

7. Dispositif selon l'une quelconque des revendications 1 à 6, dans lequel le réflecteur (3) comporte une face active concave torique.

8. Dispositif selon l'une quelconque des revendications 1 à 6, dans lequel le réflecteur (3) comprend une pluralité d'éléments de réflexion, chaque élément de réflexion comportant une face active en portion de cylindre.

9. Dispositif selon l'une quelconque des revendications 6 à 8, lequel comprend en outre un support (7) pour supporter les moyens de génération d'ultrasons (2) et chaque réflecteur (3), les moyens de génération d'ultrasons (2) étant positionnés dans une région périphérique du support (7), et chaque réflecteur (3) étant positionné dans une région centrale du support (7).

10. Dispositif selon la revendication 9, dans lequel le réflecteur (3) comporte une face active (33) en forme de tronc de cône, ledit tronc de cône étant concave ou plan ou convexe.

11. Dispositif selon la revendication 1, dans lequel les moyens de génération d'ultrasons (2) présentent une forme tubulaire et le réflecteur (3) présente une forme en tronc de cône, ledit tronc de cône étant concave, plan ou convexe, le diamètre des moyens de génération d'ultrasons (2) étant supérieur au diamètre du réflecteur (3), les moyens de génération d'ultrasons (2) et le réflecteur (3) étant agencés de sorte que :
- les axes de révolution du réflecteur (3) et des moyens de génération d'ultrasons (2) sont confondus,
- les moyens de génération d'ultrasons (2) entourent le réflecteur
- la face interne des moyens de génération d'ultrasons (2) rayonnant les ultrasons est en regard de la face active (33) du réflecteur (3).

## Patentansprüche

1. Vorrichtung zur Behandlung einer Augenerkrankung, umfassend einen Ring (1), aufweisend ein proximales Ende (11), das bestimmt ist, mit einem Auge (4) eines Patienten im Kontakt zu sein, und ein distales Ende (12), das bestimmt ist, Ultraschallerzeugungsmittel (2) aufzunehmen, wobei die Vorrichtung ferner mindestens einen Reflektor (3) umfasst, der eine konkave aktive Fläche (33) gegenüber den Ultraschallerzeugungsmitteln (2) einschließt, um den von den Erzeugungsmitteln (2) erzeugten Ultraschall in einer Region (41) des Auges (4) des Patienten zu reflektieren und zu fokussieren, und die Ultraschallerzeugungsmittel (2) mindestens einen Wandler umfassend, der eine vordere Fläche (22) aufweist, um den Ultraschall zu senden, wobei sich die vordere Fläche (22) jedes Wandlers parallel zur Drehachse (A-A') des Rings (1) erstreckt.

2. Vorrichtung nach Anspruch 1, wobei die Ultraschallerzeugungsmittel (2) einen einzigen rohrförmigen Wandler umfassen.

3. Vorrichtung nach Anspruch 1, wobei die Ultraschallerzeugungsmittel (2) eine Vielzahl von Wandlern umfassen, wobei die Wandler auf den Mantellinien eines Rohrs positioniert sind.

4. Vorrichtung nach Anspruch 3, wobei jeder Wandler eine Zylinderabschnittsform aufweist.

5. Vorrichtung nach Anspruch 3, wobei jeder Wandler eben ist.

6. Vorrichtung nach einem der Ansprüche 1 bis 5, welche ferner eine Halterung (7) umfasst, um die Ultraschallerzeugungsmittel (2) und jeden Reflektor (3) zu halten, wobei die Ultraschallerzeugungsmittel (2) in einer zentralen Region der Halterung (7) positioniert sind, und jeder Reflektor (3) in einer peripheren Region der Halterung (7) positioniert ist.

7. Vorrichtung nach einem der Ansprüche 1 bis 6, wobei der Reflektor (3) eine torische konkave aktive Fläche aufweist.

8. Vorrichtung nach einem der Ansprüche 1 bis 6, wobei der Reflektor (3) eine Vielzahl von Reflexionsmitteln umfasst, wobei jedes Reflexionselement eine aktive Zylinderabschnittsfläche aufweist.

9. Vorrichtung nach einem der Ansprüche 6 bis 8, welche ferner eine Halterung (7) umfasst, um die Ultraschallerzeugungsmittel (2) und jeden Reflektor (3) zu halten, wobei die Ultraschallerzeugungsmittel (2) in einer peripheren Region der Halterung (7) positioniert sind, und jeder Reflektor (3) in einer zentralen Region der Halterung (7) positioniert ist.

10. Vorrichtung nach Anspruch 9, wobei der Reflektor (3) eine kegelstumpfförmige aktive Fläche (33) umfasst, wobei der Kegelstumpf konkav oder eben oder konvex ist.

11. Vorrichtung nach Anspruch 1, wobei die Ultraschallerzeugungsmittel (2) eine rohrförmige Form aufweisen und der Reflektor (3) eine kegelstumpfförmige Form aufweist, wobei der Kegelstumpf konkav, eben oder konvex ist, wobei der Durchmesser der Ultraschallerzeugungsmittel (2) größer als der Durchmesser des Reflektors (3) ist, wobei die Ultraschallerzeugungsmittel (2) und der Reflektor (3) derart angeordnet sind, so dass:
- die Drehachsen des Reflektors (3) und der Ultraschallerzeugungsmittel (2) zusammenfallen,
- die Ultraschallerzeugungsmittel (2) den Reflektor umgeben,
- sich die Innenfläche der Ultraschallerzeugungsmittel (2), welche den Ultraschall senden, gegenüber der aktiven Fläche (33) des Reflektors (3) befindet.

## Claims

1. A device for treating an ocular pathology, comprising a ring (1) having a proximal end (11) intended to be in contact with a patient's eye (4) and a distal end (12) intended to receive ultrasound generating means (2), the device further comprising at least one reflector (3) including an active concave face (33) facing the ultrasound generating means (2) to reflect and focus the ultrasounds generated by the generating means (2) in a region (41) of the patient's eye (4), and the ultrasound generating means (2) comprising at least one transducer having a front face (22) to radiate the ultrasounds, the front face (22) of each transducer extending parallel to the axis of revolution (A-A') of the ring (1).

2. The device according to claim 1, wherein the ultrasound generating means (2) comprise a single tubular transducer.

3. The device according to claim 1, wherein the ultrasound generating means (2) comprise a plurality of transducers, said transducers being positioned on generating lines of a tube.

4. The device according to claim 3, wherein each transducer is in the shape of a cylinder portion.

5. The device according to claim 3, wherein each transducer is planar.

6. The device according to any one of claims 1 to 5, which further comprises a support (7) for supporting the ultrasound generating means (2) and each reflector (3), the ultrasound generating means (2) being positioned in a central region of the support (7) and each reflector (3) being positioned in a peripheral region of the support (7).

7. The device according to any one of claims 1 to 6, wherein the reflector (3) comprises an active concave toroid face.

8. The device according to any one of claims 1 to 6, wherein the reflector (3) comprises a plurality of reflecting elements, each reflecting element comprising an active face in the shape of a cylinder portion.

9. The device according to any one of claims 6 to 8, which further comprises a support (7) for supporting the ultrasound generating means (2) and each reflector (3), the ultrasound generating means (2) being positioned in a peripheral region of the support (7) and each reflector (3) being positioned in a central region of the support (7).

10. The device according to claim 9, wherein the reflector (3) comprises an active face (33) in the shape of a truncated cone, said truncated cone being concave or planar or convex.

11. The device according to claim 1, wherein the ultrasound generating means (2) are of tubular shape and the reflector (3) is of truncated cone shape, said truncated cone being concave, planar or convex, the diameter of the ultrasound generating means (2) being larger than the diameter of the reflector (3), the ultrasound generating means (2) and the reflector (3) being arranged so that:
- the axes of revolution of the reflector (3) and of the ultrasound generating means (2) coincide,
- the ultrasound generating means (2) surround the reflector,
- the inner face of the ultrasound generating means (2) radiating the ultrasound waves faces the active face (33) of the reflector (3).
